# EUROPEAN PATENT SPECIFICATION

(11) **EP 4 243 613 B1**
(45) Date of publication and mention of the grant of the patent: **18.02.2026**
(21) Application number: 21810986.6
(22) Date of filing: 11.11.2021
(51) Int. Cl.: A01M 21/04

(54) **METHOD AND APPARATUS FOR POSITIONING OF LASER BEAM**
VERFAHREN UND VORRICHTUNG ZUR POSITIONIERUNG EINES LASERSTRAHLS
PROCÉDÉ ET APPAREIL DE POSITIONNEMENT D'UN FAISCEAU LASER

(30) Priority: 11.11.2020 LV 200076
(43) Date of publication of application: 20.09.2023
(73) Proprietor: Weedbot, Sia, Riga 1050 (LV)
(72) Inventor: JASKO, Janis, 2121 Salaspils (LV); KOSTROMINS, Andrejs, 3003 Jelgava (LV); OSADCUKS, Vitalijs, 3017 Jelgavas nov. (LV); PECKA, Aldis, 3003 Jelgavas novads (LV); SUDARS, Kaspars, 1046 Riga (LV); PIRS, Vilnis, 3101 Tukums (LV)
(74) Representative: Gill Jennings & Every LLP
(86) International application number: PCT/EP2021/081396
(87) International publication number: WO 2022/101346

(56) References cited:
- EP-A1- 3 545 760
- JP-A- 2006 136 923
- US-A1- 2015 075 066

## Description

### FIELD OF THE INVENTION

Invention applies to the field of optics, specifically to laser technologies, more specifically to industrial lasers, particularly to laser beam positioning. The invention has particular application for the laser-treatment of weeds or other unfavourable plants.

### BACKGROUND TO THE INVENTION

There are several methods to position lasers, for example, robotic arms with multiple degrees of freedom, manipulators, CNC type mechanisms, rotating prisms, or two perpendicular mirrors - galvanometer scanners (galvo scanners). There are also solutions available to track moving objects using image recognition software based on neural network algorithms, including for purpose to position lasers on them. A few examples of such technologies are presented in European patent publication No. 1098563, US patent publication No. 6,795,568 and US patent publication No. 10,6819052. Nevertheless these technologies don't provide fast enough fulfilment of all tasks: recognition of defined objects, object segmentation, and treatment with a laser in the whole selected area, so it is necessary to have additional means to detect speed of the moving object, for example, radars, lidars or speed wheels. These additional components make the system too complicated and increase costs. For example, detection and segmentation of objects in an image which is 500x500 pixels or larger, usually takes several tens to several hundreds of milliseconds, so without speed control and speed correction it is practically impossible to make real-time laser treatment, because object would be in a different place after this time period.

To avoid the need to measure and compensate for the relative displacement of an object relative to the camera and / or laser, a technique has been developed that allows a laser beam to process a moving object or a stationary object with a moving device, i.e. laser and laser beam, respectively, with millimeter accuracy or better. An obvious solution to the above problem would be to significantly increase the moving speed of the laser, which is not cost-effective, as it would require more than 100 times more powerful laser to get the same treatment effect, which would be technologically complex and financially inefficient.

Important art in the field is for example US 2015/075066 A1.

### SUMMARY OF THE INVENTION

The invention comprises the method and the apparatus as described in independent claims 1 and 9 and in their dependent claims. Other aspects not covered by this scope may be described herein. In accordance with an aspect there is provided a method for positioning a laser, which includes following steps:
a. image acquisition;
b. identification of objects of interest in the image;
c. division of the laser-treated area into treatment lines;
d. calculation of the coordinates of the lines in physical space;
e. treatment (e.g. processing) of lines with a laser-generated laser beam;
preferably characterized in that one cycle with the step mentioned above is realized in 25 milliseconds or less.

The above problems or work tasks are solved by creating a method for laser positioning and processing of objects of interest with a laser beam, which includes image acquisition step, followed by identification of objects of interest in the image. The object of interest may be weed or other unfavorable non-agricultural plant or part of a plant. The method is characterized by the division of the area to be processed into lines, followed by the calculation of the positions of the lines in space and finally by the processing of the lines with laser beam. As a result, one cycle with the above-mentioned steps is realized in up to 25 milliseconds. In some cases, one cycle of the steps (a) to (e) may take less than 25 milliseconds.

In this way, the present invention allows for laser treatment of objects that are moving relative to the laser providing the laser-generated beam, with a precision of the order of ~1mm at acceptable movement speeds. Preferably, the method comprises (e.g. iteratively) performing a plurality of cycles of the steps (a) to (e) as the laser and objects of interest are in motion relative to each other. For instance, the method may comprise moving the laser relative to the object(s) of interest during and/or between each cycle of steps (a) to (e). In other words, on completion of one cycle (a) to (e), the process starts again with an acquisition of a further image. Within each cycle, identified treatment line(s) are treated by the laser beam. In this way, a plurality of images may be acquired and processed in order to provide laser treatment of objects of interest whilst the objects of interest and the laser are in motion relative to each other. For example, the method of the invention may be implemented on an apparatus that is towed by a vehicle (e.g. a tractor) in an agricultural field in order to provide laser treatment of undesirable weeds in the field while the apparatus moves relative to the objects of interest. In such examples the method may comprise moving the laser (and any camera or further components of the apparatus) during and/or between each cycle of steps (a) to (e). It is also envisaged that the invention may be used in a "static" mode in which the objects of interest and the laser are not in relative motion.

The laser-treated area is typically the area, or portion, of the image (or in some instances the whole image) that corresponds to the region that is desired to be treated by the laser, dependent on the particular application. For example, the laser-treated area may be a whole object of interest or multiple objects of interest (e.g. weeds), or only part of one or more objects of interest. In some examples, the laser-treated area may be a narrow "single line" (for example 5 to 10 pixels wide, or even in some cases less than 5 pixels wide) spanning across the width of the image, corresponding to approximately the same line width of the laser beam.

Preferably, in step e, only a subset of lines (e.g. treatment lines) are treated with the laser beam. In particularly preferred embodiments, in step (e) only a single (e.g. exactly one) line is treated with the laser beam. Thus, in contrast to state of the art processes which may attempt to treat the whole of each identified object of interest in the acquired image, the present invention preferably treats sections of object(s) of interest arranged along a single line with the laser beam within one cycle of the steps a to e. This advantageously reduces the time taken to perform a cycle of steps a to e, typically to 25 milliseconds or less. In this way, a plurality of cycles of the steps a to e can be performed in order to treat the objects of interest as the laser and objects of interest are in motion relative to each other.

The line or lines treated by the laser in step (e) may comprise one or more segments (which may also be referred to as "laser treatment regions") dependent on the position of the objects of interest in the acquired image. Each segment is typically a region of the single line that overlaps with an identified object of interest for laser treatment. The treatment line(s) are typically orientated in a cross direction to a direction of relative motion between the laser (e.g. an apparatus carrying the laser) and objects of interest, preferably being arranged substantially perpendicular relative to the direction of relative motion (e.g. angled at at least 60 degrees relative to the direction of relative motion, more preferably at least 75 degrees). Typically, the treatment line(s) have a dimension (e.g. along the direction of relative motion) smaller than a dimension of the objects of interest in the image (e.g. less than 10 pixels). However, in general the treatment line(s) may have any orientation, for example along the direction of motion

The completion of one cycle of the steps (a) to (e) within 25 milliseconds advantageously allows for the present invention to treat the objects of interest (e.g. weeds) while continuously moving at a speeds of up to 300m/hr (a typical average speed for manual weeding) with a ~2mm spatial error, which is deemed acceptable. Thus, preferably the method comprises moving the laser relative to the object(s) of interest by approximately 300m/hr (e.g. between 100 to 3000 m/hr, more preferably between 200 and 500m/hr, more preferably between 200 and 400 m/hr).

Step b comprises the identification of objects of interest (e.g. weeds) in the acquired image. The image is typically an image of the ground (e.g. in an agricultural field). The acquired image typically represents a size of 50mm x 50mm of the ground surface or larger. The image is preferably a two-dimensional image. As will be explained in more detail below, the identification of the objects of interest typically comprises detection of green pixels in the acquired image and instance segmentation of objects within the acquired image. The instance segmentation is typically performed using a machine learning algorithm such as a neural network. A preferred machine learning algorithm is a convolutional neural network.

The machine learning algorithm is configured to differentiate between objects that are not intended for laser treatment (e.g. agricultural crops) and the objects of interest that are desired to be treated by the laser (e.g. weeds or other unfavourable non-agricultural plants or parts of a plant). In particular, the machine learning algorithm is typically configured to identify objects that are not intended for laser treatment. The machine learning algorithm is typically trained using training data comprising tagged images. For example, the training data may comprise a plurality of images that are tagged as agricultural crops or weeds.

By using a machine learning algorithm to identify objects that are not intended for laser treatment, and detection of green pixels in the acquired image, it is possible to infer that the identified green pixels that do not correspond to the identified objects that are not intended for laser treatment (e.g. agricultural crops) correspond to objects of interest for laser treatment (e.g. weeds).

Step d comprises calculation of the coordinates of the lines (e.g. treatment lines) in physical space. In other words, the coordinates of the treatment lines determined in step c are transformed from the image space (e.g. pixel coordinates) into the physical "real-world" space (e.g. in Cartesian or polar coordinates). This may be performed based on knowledge of the geometry of the camera used to acquire the image relative to the ground.

In step e, the lines (e.g. treatment lines) are treated with a laser-generated laser beam. The thermal energy provided by the laser beam preferably kills or otherwise damages the object of interest for laser treatment (e.g. weed). The laser beam is generated by a laser, with the direction and movement of the laser beam preferably being controllable by means of galvanometer. In other words, the laser beam is preferably guided by means of a galvanometer scanner. The inventors have found that the use of galvanometer scanner is particularly advantageous as this allows for the fast and precise control of the laser beam. This is especially beneficial when using high-powered lasers that are typically heavy and unwieldy to physically move.

Moreover 50mm x 50mm or greater picture is captured. Laser beam is moved by the help of galvo-scanner, where laser output beam is aligned with the camera objective at a distance of 15 cm or closer. Typically, the exit point of the laser beam and the centre of the camera lens are positioned at a distance of 15 cm or closer from each other. Minimising these distances increases accuracy and avoids errors (e.g. parallax errors) in the positioning of the laser-beam.

The identification of objects of interest in the image typically includes the following sub-steps:
b1. Detection of green pixels in the image,
b2. Instance segmentation of objects of interest,
b3. Conversion of acquired masks of instance segmentation to polygon coordinates,
b4. Acquisition of binary image, where all objects of interest are marked, (e.g. including crops), while waiting for result of green pixel detection,
b5. Acquisition of coordinates of polygons of crops to cover green pixels of crops in the binary image and leave only objects for treatment in such a way obtaining a binary image of objects of interest for laser treatment.
b6. Acquisition of coordinates of objects of interest for laser treatment from the binary image constructed in the previous step.

As discussed, the instance segmentation of objects within the image is typically performed using a machine learning algorithm such as a convolutional neural network. The instance segmentation in step b2 is typically instance segmentation of objects of interest not intended for laser treatment (e.g. agricultural crops). In other words, preferably the machine learning algorithm only identifies objects of interest that are not intended for laser treatment (e.g. crops such as carrots). By identifying only one class, the cycle speed is increased. However, in alternative embodiments, a multiple class machine learning algorithm (e.g. CNN) may be used to identify both objects of interest intended for laser treatment and those not intended for laser treatment.

In steps b3 and b4, the coordinates of polygons of objects not intended for laser treatment (e.g. crops) and optionally objects of interest for laser treatment (e.g. weeds) are acquired and marked on a binary image while waiting for the result of the green pixel detection. In other words, the detection of green pixels in step b1 and the steps of b2 to b4 are typically performed substantially simultaneously, for example in parallel CPU processing threads.

In step b5, a binary image of objects of interest for laser treatment (e.g. weeds) is acquired. This is achieved by combining the result of the green pixel detection in step b1 (e.g. a binary mask of green pixels in the image) with the binary image obtained from the instance segmentation. The identified objects not intended for laser treatment may be subtracted from the binary mask of green pixels in the image, thereby obtaining a binary image of objects of interest for laser treatment (e.g. weeds). In step b6 the coordinates (e.g. polygon coordinates) of the objects of interest for laser treatment may then be acquired.

In a preferred embodiment, the identification of objects of interest in the image comprises: b1. detecting green pixels in the image and acquiring a binary mask of the green pixels; b2. performing instance segmentation of objects of interest not intended for laser treatment and obtaining a binary mask of the objects of interest not intended for laser treatment; and b3 generating a binary mask of the objects of interest for laser treatment by subtracting the binary mask obtained in step b2 from the binary mask obtained in step b1.

In preferred embodiments, the detection of green pixels is performed in a portion of the acquired image (rather than across the entire image). Although the whole acquired image may be analysed for instance segmentation and identification of objects of interest, only the green pixels of the objects of interest for laser treatment within the identified treatment lines are required to be treated by the laser beam. This advantageously increases processing speed. In some embodiments, only a portion of the image may be analysed by the CNN in order to further increase processing speed. However, typically such a portion is typically required to be at least 1.5x, preferably at least 2x larger than the size of an object not intended for laser treatment (e.g. a crop).

Division of the area intended for laser treatment in to treatment lines typically includes the following sub-steps
c1. Construction of theoretical treatment lines with predefined distance in pixels,
c2. Calculation of intersection points with the polygons of polygons of objects of interest for laser treatment (e.g. weeds),
c3. Construction of treatment line grid from intersection pairs obtained in the previous step.

In step c1, one or more theoretical treatment lines (which may also be referred to as "laser processing lines" herein) are constructed with predefined distance in pixels. The theoretical treatment line(s) typically have a predetermined relationship with the acquired image, dependent on the geometrical relationship between the camera and the laser. In preferred embodiments, a theoretical treatment line spans the width of the image. The theoretical treatment lines are typically orientated in a cross direction to a direction of relative movement between the laser and objects of interest. In preferred embodiments, a theoretical treatment line has a dimension (e.g. along the direction of relative motion) smaller than a dimension of the objects of interest. This may be a dimension of 10 image pixels or less, preferably 5 image pixels or less and more preferably 1 pixel.

The theoretical treatment line(s) are typically located within the laser-treated area. In other words, the number of theoretical treatment lines is typically dependent on the size of the laser-treated area. In alternative embodiments, the treatment lines may be determined using techniques such as detection of pixel brightness, for example with the treatment lines corresponding to areas of pixel brightness above a predetermined threshold.

In some embodiments, the division of the area intended for laser treatment comprises constructing one or more theoretical treatment lines with predefined distance in pixels, and determining region(s) of overlap between the theoretical treatment line(s) and identified object(s) of interest for laser treatment.

In addition, calculation of each treatment line coordinates in the physical space include transformation of each line from coordinate system in pixels to coordinate system in metric units in physical space. Line treatment with laser generated laser beam include reception of converted line in metric coordinates and treatment of object of interest. Angular coordinate systems (e.g. polar coordinates) in physical space may be used, as well as Cartesian coordinate systems.

The laser beam is preferably guided by means of a galvanometer scanner. This is particularly advantageous as it allows fast and precise control of the laser beam, in contrast to alternative mechanisms such as gimbals or turntables. The laser beam is typically a visible light laser beam with a wavelength of 400-500nm and a power density of at least 15kW/cm^2.

Preferably, the images are acquired by a visible light camera with a speed at least 70 frames per second, even better 200 frames per second, even more better 500 frames per second with a resolution of 0.3 Megapixels or greater. Object of relevance is preferably a plant or part of the plant and it is typically detected using object detection based on neural network algorithms.

Moreover in each step, preferably only a single line is treated by a visible light laser beam, typically in the wavelength of the 400-500 nm and preferably having a power density 15kW/cm^2 or more. A power density of 15Kw/cm^2 or more has been found to provide enough energy to kill or otherwise damage the objects of interest for laser treatment such as weeds.

In accordance with a second aspect of the invention there is provided an apparatus for positioning a laser beam, comprising:
a camera configured to capture an image;
a laser configured to generate a laser beam;
a laser beam positioning device operably coupled to the laser and configured to position the laser; and
one or more processors configured to perform the steps of the first aspect of the invention discussed above.

Preferably, the laser beam positioning device is a galvanometer scanner.

In accordance with a further aspect of the invention there is provided an apparatus for positioning a laser beam, comprising:
a camera configured to capture an image;
a computing device configured to identify the object of interest in the obtained image;
a laser configured to generate the laser beam;
a laser beam positioning device operably coupled to the laser and configured to position the laser so as to position the laser beam itself;
in addition, the computing device is connected to the laser and the laser beam positioning device which according to the identified object of interest, triggers the generation of the laser beam and positions the laser beam through the laser positioning device so that the laser beam reaches the object of interest,
characterized in that the laser beam positioning device is a galvanometer scanner.

Thus, the apparatus of the present invention provides all of the advantages discussed above in relation to the method of the first aspect of the invention.

The apparatus contains several basic elements. It contains a camera configured to obtain an image, a computing device configured to identify the object of interest in the acquired image, and a laser configured to generate a laser beam.

The apparatus also comprises a laser beam positioning device appropriately coupled to the laser and configured to position the laser, thereby positioning the laser beam itself. The apparatus is characterized in that a galvo scanner is used as the laser beam positioning device. In addition, the computing device is connected to the laser and the laser beam positioning device in order to give start command for the generation of the laser beam according to the identified object of interest and to position the laser beam through the laser positioning device so that the laser beam reaches the object of interest. In addition, the computing device includes a neural network node configured to recognize an object of interest, such as a plant or part of a plant.

Typically, the laser is configured to generate a laser beam with a wavelength of 400-500 nm and a power density of at least 15 kW / cm^2. These are suitable parameters to damage and/or kill weeds and other undesirable plants.

Preferably, the laser is configured to emit laser beam in the wavelength of 445 nm and power density of 16,7 kW/cm^2.

Innovative approach described in the previous sections eliminates the need for movement speed measurement and/or movement measurement and compensation ensuring treatment for the whole working area. Treatment region for each treatment step is reduced significantly by this approach. By this we mean that the laser receives a treatment command only for a single line (e.g. a single theoretical treatment line) (or a limited number of multiple lines to maintain claimed accuracy) instead of all lines in array as it would be if standard treatment approach would be used for the whole working area at once. Single line can be treated in a few milliseconds not exceeding 20 milliseconds (depending on the total line length and laser power), which introduces only a millimeter or less (or a different predetermined maximum allowed spatial error displacement) as relative displacement measured from starting position. Next image is acquired after the line is treated by the laser, objects are identified and segmentation polygons are calculated and next treatment coordinates are sent to the laser. Thus dividing the working area in a single line tasks ensuring ability to perform high accuracy treatment of one millimeter or less while objects are in motion relative to the laser module.

An additional light source, such as a flash or other light source, may be installed to provide a sufficient amount of light.

To eliminate the need for stereo disparity compensation between the laser beam exit point and the camera lens, the camera center and the laser beam exit point should be located 15 cm or closer.

Typically, the apparatus according to the invention is configured to be mounted on or integrated in a vehicle (e.g. a tractor or any other suitable manned or autonomous vehicle) or a unit (e.g. a trailer) configured to be attached to a vehicle. Preferably the apparatus (and any vehicle to which the apparatus is mounted or attached) is configured to move the laser relative to the object(s) of interest by approximately 300m/hr (e.g. between 100 to 3,000 m/hr, more preferably between 200 and 500m/hr, more preferably between 200 and 400 m/hr).

In accordance with a third aspect of the present invention, there is provided a method, performed in a laser treatment apparatus, comprising: (a) acquiring an image; (b) identifying one or more objects of interest for laser treatment within the acquired image; (c) selecting a laser treatment area of the acquired image and dividing the laser treatment area into one or more laser processing lines; (d) determining one or more laser treatment regions within the laser processing line(s) based on the identified object(s) for laser treatment; and (e) treating the laser treatment region(s) with a laser beam.

Thus, the method of the third aspect provides all of the advantages discussed above. The method of the third aspect may comprise any of the preferable or optional features discussed above with reference to the preceding aspects of the invention and offer corresponding advantages. In particular, by dividing the laser treatment area into one or more laser processing lines, and treating the laser treatment region(s) with the laser beam, the method allows objects of interest to be laser-treated while the laser apparatus and objects of interest are in relative motion, with a precision of the order of approximately 1mm at acceptable movement speeds.

The laser treatment area is typically the area, or portion, of the image (or in some instances the whole image) that corresponds to the region that is desired to be treated by the laser, dependent on the particular application, as has been described above.

Typically, each laser processing line has a dimension that is smaller than a dimension of the objects of interest for laser treatment (e.g. in image coordinates). In this way, the method allows for faster processing of the acquired image. In some examples, the laser processing lines have a dimension in the direction of relative motion of 10 pixels or less, preferably 5 pixels or less, more preferably 1 pixel.

Preferably, the laser treatment region(s) within a subset of the laser processing lines are treated with the laser beam in step (e). In this way, the speed with which the cycle of steps (a) to (e) is performed is increased, as only a subset, or portion, of the area corresponding to the image is treated with the laser beam. This is in contrast to prior art methods which as described above may attempt to treat each part of the image. In particularly preferred embodiments, the laser treatment regions within exactly one (e.g. only a single) processing line are treated with the laser beam in step (e). In this way, the processing speed may be maximised. In preferred embodiments the sequence of steps (a) to (e) may be performed in 25ms or faster.

Typically, each laser processing line is elongate and orientated in a cross direction to a direction of relative movement between the apparatus and the objects of interest. Preferably, the positions of the laser processing lines are predetermined for each acquired image.

The method preferably comprises (e.g. iteratively) performing a plurality of the cycles of the steps (a) to (e) as the laser treatment apparatus and the objects of interest for laser treatment move relative to each other. For instance, the method may comprise moving the laser treatment apparatus relative to the object(s) of interest during and/or between each cycle of steps (a) to (e). Preferably, the method comprises moving the apparatus relative to the object(s) of interest by approximately 300m/hr (e.g. between 100 to 3000 m/hr, more preferably between 200 and 500m/hr, more preferably between 200 and 400 m/hr).

The method preferably comprises converting the determined laser treatment region(s) into physical space coordinates for treatment by the laser beam.

In preferred embodiments, the laser beam is generated by a laser and is directed by a galvanometer scanner.

Preferably, the identifying one or more objects for laser treatment within the acquired image comprises identifying objects of interest within the acquired image using a machine learning algorithm, preferably a neural network. The machine learning algorithm may be configured to identify objects of interest that are not intended for laser treatment (e.g. agricultural crops). Typically, the method also comprises identifying green pixels within the image or a portion of the image. By also identifying green pixels within the image, it can be inferred that the green pixels that do not correspond to the objects of interest that are not intended for laser treatment instead correspond to objects of interest that are intended for laser treatment (e.g. weeds). A binary mask of objects of interest for laser treatment may be generated as described above.

Step (d) of the method comprises determining one or more laser treatment regions within the laser processing line(s) based on the identified object(s) for laser treatment. Typically, this step comprises determining the region(s) where the object(s) of interest for laser treatment overlap with the laser processing line(s). A single laser processing line may comprise exactly one laser treatment region, or could comprise two or more laterally spaced laser treatment regions collinearly arranged along the laser processing line dependent on the relative arrangement of the laser processing lines and the objects of interest.

In accordance with a fourth aspect of the invention there is provided a laser treatment apparatus, comprising: a camera configured to acquire an image; a laser configured to generate a laser beam; a laser beam positioning device configured to direct the laser beam; and an image processing unit configured to perform the steps of: (a) receive an image acquired by the camera; (b) identify one or more objects of interest for laser treatment within the acquired image; (c) select a laser treatment area of the acquired image and divide the laser treatment area into one or more processing lines; (d) determine one or more laser treatment regions within the laser processing line(s) based on the identified object(s) for laser treatment; and (e) control the laser and laser beam positioning device so as to treat the laser treatment regions(s) with the laser beam.

The laser treatment apparatus therefore provides all of the advantages discussed above. The image processing unit may be configured to perform any of the steps discussed above. As such, the image processing unit may comprise or be configured to perform any of the preferable or optional features discussed above with reference to the preceding aspects of the invention and offer corresponding advantages.

Preferably, the image processing unit is further configured to control the laser and laser beam positioning device such that the laser treatment region(s) within a subset of the processing lines are treated in step (e). Preferably, the image processing unit is further configured to control the laser and laser beam positioning device such that the laser treatment region(s) within (e.g. only) exactly one processing line are treated within step (e).

Preferably, the laser beam positioning device is a galvanometer, although other devices are envisaged such as gimbals or turntables to which the laser is mounted. Typically, the laser is configured to generate a laser beam with a wavelength in the region of 400-500nm and a power density of at least 15kW / cm^2.

Typically, the laser treatment apparatus is configured to be mounted on or integrated in a vehicle (e.g. a tractor or any other suitable manned or autonomous vehicle) or a unit (e.g. a trailer) configured to be attached to a vehicle. Preferably the apparatus (and any vehicle to which the apparatus is mounted or attached) is configured to move the laser relative to the object(s) of interest by approximately 300m/hr (e.g. between 100 to 3,000 m/hr, more preferably between 200 and 500m/hr, more preferably between 200 and 400 m/hr).

In accordance with a first example there is provided technology for laser positioning consisting of the following stages: a. acquisition of an image; b. identification of the objects of interest in the image; c. division of the area to be processed into processing lines; d. calculation of the positions of the lines to be processed in the space; e. processing of the lines with a laser; differs by the fact that the whole process takes less than 25 milliseconds.

Preferably, the technology is characterised by the fact that the size of the obtained image in nature is 50 mm x 50 mm or more.

Preferably, the technology is characterised by the fact that the laser beam is directed by means of the mechanism of a galvoscanner.

Preferably, the technology is characterised by the fact that the output point of the laser and the center of the camera are at a distance of 15 cm or less.

Preferably, the technology is characterised by the fact that a. To obtain an image a visible light camera is used at a speed of at least 500 frames per second and with a resolution of 0.3 megapixels or more; b. The object of interest is a plant or a part of a plant and is identified using a recognition algorithm based on neuron network technologies; c. only one line is processed in a single session; d. The line is processed using a visible light laser with a wavelength of 400 to 500 nm and a power density of at least 15 kW/cm^2.

In accordance with a second example there is provided equipment for processing an object with a laser, consisting of the following parts: a. camera; b. computer; c. laser; differs by the fact that it can acquire an image, identify the object of interest in the image, and process one line with a laser so that the whole process takes less than 25 milliseconds.

### BRIEF DESCRIPTION OF THE DRAWINGS

The invention will be described with reference to the appended drawings, in which:
Figure 1 is a principal scheme of the technology;
Figure 2 is a principal scheme of the equipment;
Figure 3 is a flow chart setting out the steps of a method according to an embodiment of the invention;
Figures 4(a) to 4(c) illustrate the processing of sequence of images according to an embodiment of the invention;
Figure 5 schematically illustrates an apparatus according to an embodiment of the invention; and
Figure 6(a) to 6(c) schematically illustrate the construction of laser treatment regions according to an embodiment of the invention.

### DETAILED DESCRIPTION

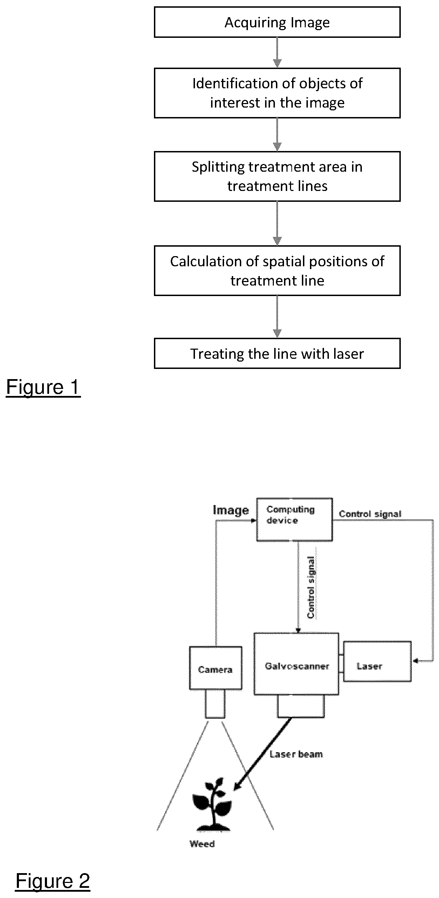

In one example, the technology consists of several basic elements shown in Figure 1.

An example will now be described. In order to exclude the need to use the measurement and compensation of the displacement speed and/or displacement, while ensuring the required processing area, the area to be processed in a single laser run is significantly reduced. This means that a command is sent to the laser to process only one line (or some other maximum permissible number of lines to fit within the predefined fault limits), not an entire array of lines, as it would be in the case of the standard approach. Processing of one line may be performed in a unit of time ranging from a few milliseconds to teen milliseconds (depending on the length of the line and the power of the laser), which results in a relative difference with respect to the original position within only a millimeter or fraction of a millimter (or some other predefined maximum permissible error). After completing processing of the line, the next image is obtained, the site is identified and segmented and the coordinates of the next line to be processed are sent to the laser. Thus, by dividing the area to be processed into tasks of single lines, it is possible to process objects in motion with a laser with the accuracy of a millimeter or even more precisely.

In one example, the equipment consists of several main elements shown in Figure 2.

In order to ensure a sufficient amount of light, an additional light source, such as a flash or other lighting fixture, may be installed.

In order to exclude the need to compensate for the perspective between the output point of the laser and the camera lens, the center of the camera and the output point of the laser should be located at a distance of 15 cm from each other or less.

In another example of application, the invention may be realized in the following manner. First, the camera acquires an image of the furrow area and then it is saved in RAM or other fast memory on the on-board computing device. The main thread of the computing device program crops a narrow horizontal area across the width of the acquired image and passes this area to open source program for detecting green pixels in the on-board computing device's parallel CPU thread. Then the main thread of the computing device program passes the original acquired image to a pre-trained convolutional neural network model for agricultural crop instance segmentation. The obtained instance segmentation masks are converted into polygon coordinates in parallel CPU threads. The main thread of the computing device program waits for the result from the green pixel detection program and obtains a binary mask with all green vegetation, including agricultural crops, marked. The coordinates of the agricultural crop instance polygons are used to subtract the green areas of the agricultural crops in the binary mask of the green plants and leave only the green areas of the weeds, thus obtaining the binary mask of the weeds. The main thread of the computing device program then obtains the coordinates of the weed area bounding polygons from the weed binary image and constructs laser processing path lines with a certain step in pixels and determines the intersections with the weed bounding polygon. From the obtained intersection pairs, the main program thread constructs the laser processing lines and each line is converted from the image coordinate system in pixels to the metric coordinate system of the galvo scanner area to be processed on furrow. The main thread of the computing device program puts one converted line to a queue so that the parallel CPU thread can send it to the galvo scanner using the User Datagram Protocol UDP. The galvo scanner receives the treatment line in metric coordinates and processes the weed area by positioning the laser beam as requested.

The above example may be described as follows. First the image of the area to be processed in the furrow is obtained with a camera and saved in RAM or other faster access memory of the board computer of the equipment. The software flow of the main board computer from the acquired image cuts a narrow horizontal bar across the entire width of the image and transfers this pixel bar to the open code software for determining the green pixels in the parallel CPU flow of the board computer. The main software of the board computer passes the original image to a previously trained convolution neural network model for segmenting crop instances. The resulting instance segmentation masks are converted to the coordinates of the site in parallel CPU flows. The main board computer software flow waits for the result from the detection program of green pixels and obtains a binary image marking all green plants, including crops. The site coordinates of crop instances are used to conceal green areas of crops in the binary image of green plants, and to leave only the green areas of weeds, thus obtaining a binary image of the weeds. The software flow of the main board computer retrieves from a binary image of the weeds the coordinates of the outline sites of weeded areas. The software flow of the main board computer designs imaginary processing lines with a specified step in pixels and determines intersections with outline sites of weeds. From the pairs of resulting intersections the main software flow designs the processing lines and converts each line of the image from pixels of the coordinate system to the metric coordinate system of the area to be processed on site. The software flow of the main board computer places one converted line in a row so that the parallel CPU flow sends it to a laser executing device using the user software protocol UDP. The laser receives the metric coordinates of the lines to be processed and processes the area of weeds.

It is possible to use described technology for different purposes, for example but not limited to: engraving, marking (e.g. labelling), cutting, elimination (e.g. destruction) of weeds or other harmful organisms. Technology allows to treat with laser moving objects or treat stationary objects while the laser is moving, with a precision of millimeter or even more precise.

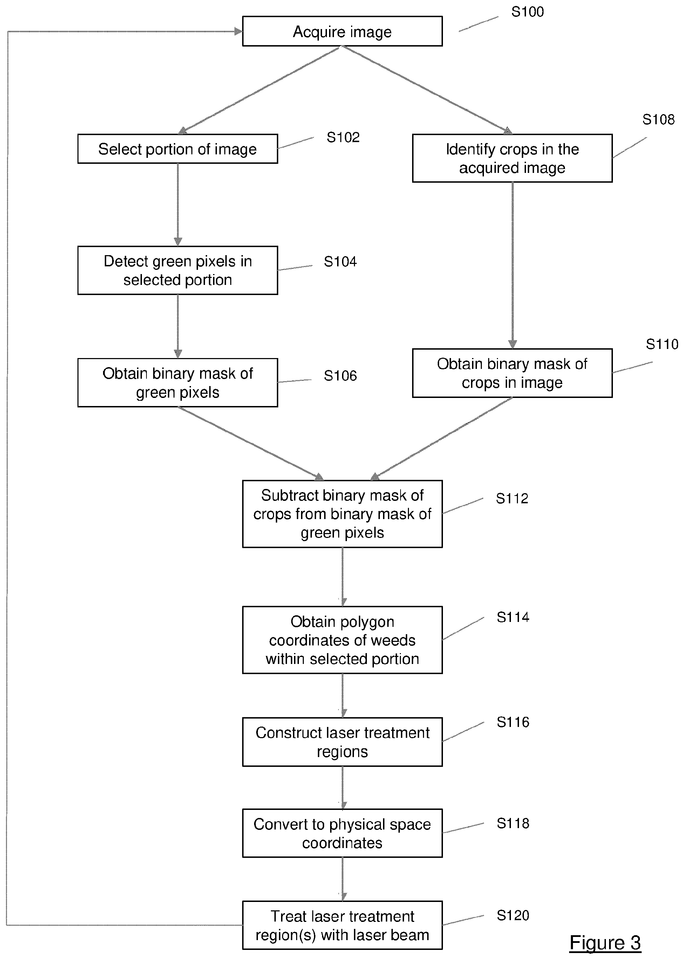

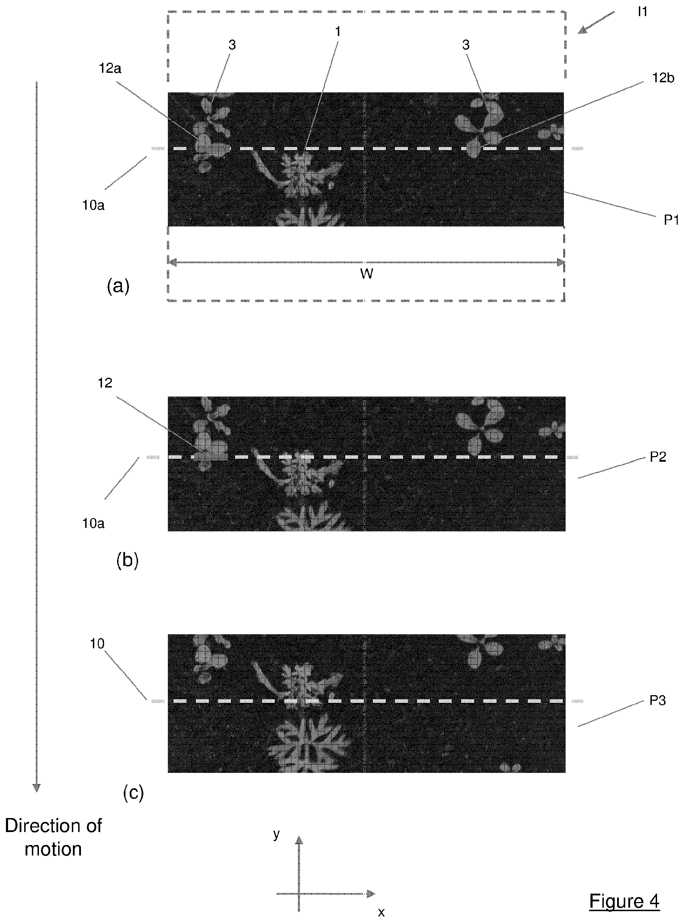

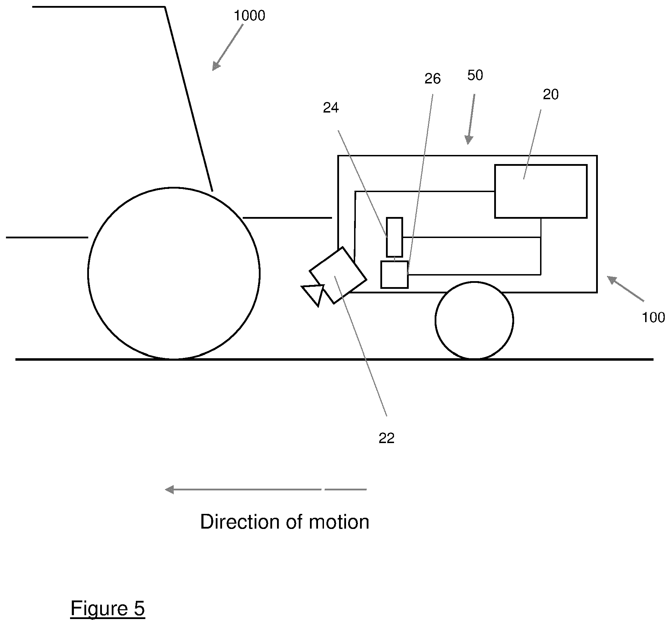

Figure 3 is a flowchart showing the main steps in a method according to an embodiment of the invention. These steps will now be explained with reference to Figures 4(a) to 4(c) as well as Figure 5, which schematically illustrates a laser treatment apparatus 50 according to an embodiment of the invention. As shown in Figure 3, the apparatus 50 comprises a camera 22, a laser 24, a laser beam positioning device 26 and an image processing unit 20. The image processing unit 20 is in logical communication with the camera 22, the laser 24 and the laser positioning unit 26. In this embodiment, the laser positioning unit 26 is in the form of a galvanometer scanner that comprises one or more mirrors configured to direct the laser beam produced by the laser 24. The camera is preferably a visible light camera with a speed of at least 70, preferably 200, more preferably 500 frames per second and a resolution of 0.3 megapixels or higher. The laser is preferably a visible light laser beam with a wavelength of 400-500nm and a power density of at least 15 kW / cm ^ 2.

In this example, the apparatus 50 is mounted on a trailer 100 that is towed by a tractor 1000. However, in other embodiments, it is envisaged that the apparatus 50 may be mounted on a vehicle that may be either manned or autonomous. The apparatus is towed by the tractor along the indicated direction of motion, with laser treatment of undesirable objects (e.g. weeds) occurring as the apparatus is in motion relative to the undesirable objects.

Referring back to Figure 3, at step S100, an image I1 of the ground of an agricultural field is acquired from a camera 22. The agricultural field typically comprises agricultural crops 1 (e.g. carrots) as well as undesirable plants such as weeds 3, as shown in Figure 4(a). It is desired to laser treat the weeds 3 with the laser so as to damage and/or kill them.

The image I1 preferably represents a portion of the ground having a size of 50mm x 50mm or greater and typically has a resolution of 0.3 Megapixels or greater. In one preferred example the image has a resolution of 1920x1200 pixels. The image is saved in RAM or other fast memory (although this is not essential). The following steps are then performed by the image processing unit 20 (e.g. computing device).

At step S102, a portion of the image I1 is selected for green pixel identification (although in other embodiments green pixels may be identified across the whole image). This is schematically shown in Figure 4(a), where the whole image I1 is indicated by the dashed lines, and the selected portion of the image P1 is illustrated. In this example, the selected portion P1 also corresponds to the desired laser treatment area, as this area comprises weeds for treatment. As shown, the selected portion P1 of the image extends across the whole width, W, of the obtained image I1 (although this is not essential), and is orientated in a cross direction to the direction of motion. At step S104, green pixels are detected in the selected portion P1, and at step S106, a binary mask of the green pixels in the selected portion P1 of the image is obtained. The steps S104 and S106 may be performed by an open source pixel detection algorithm.

In parallel with the detection of the green pixels, at step S108 the original full image I1 is processed to identify agricultural crops 1 in the image by instance segmentation. This is performed by a convolutional neural network that has been pre-trained for agricultural crop instance segmentation. The training data for training the convolutional neural network typically comprises a plurality of annotated images of agricultural crops of interest. At step S110, the obtained instance segmentation masks are converted into a single binary mask.

As is schematically shown in the flow diagram of Figure 1, the generation of the binary mask of green pixels in steps S102 to S106 and the generation of the polygon coordinates of agricultural crops in the image in steps S108 and S110 are performed in parallel (e.g. substantially simultaneously), for example in parallel CPU processing threads.

At step S112, the binary mask of the agricultural crop instances (obtained in step S110) are subtracted from the binary mask of green pixels obtained in step S106. This therefore generates a binary mask of the weeds (i.e. the green pixels that have not been identified as agricultural crops and are inferred to be objects of interest for laser treatment in the manner discussed above) in the selected portion P1 of the image.

In step S114, the polygon coordinates of the weeds within the selected portion of the image are obtained from the binary mask of the weeds generated in step S112.

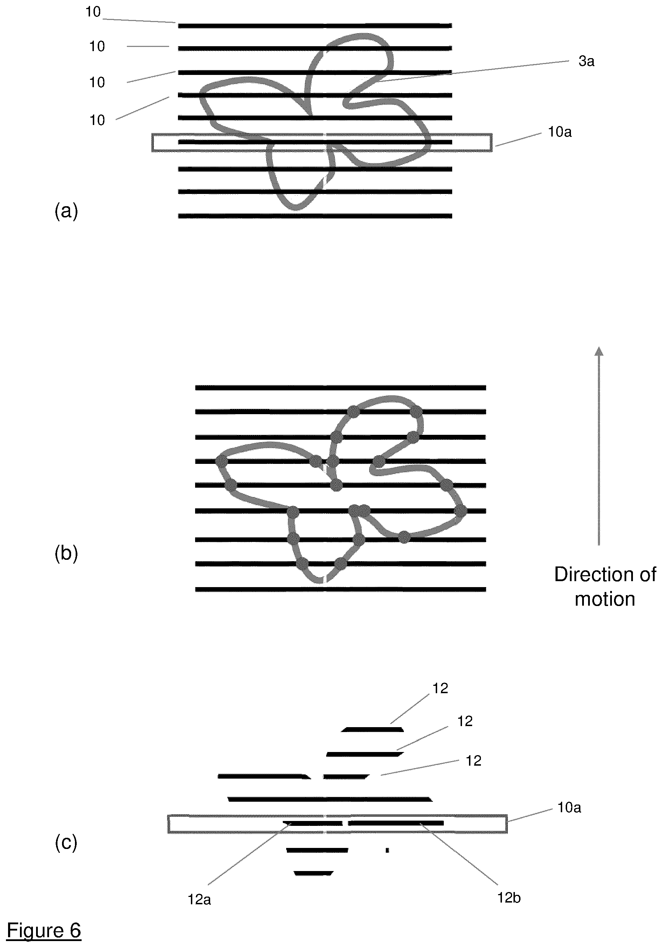

In step S116, laser treatment regions(s) 12 are constructed, which will now be explained with reference to Figures 6(a) to 6(c). This is performed by dividing the laser treatment area into a plurality of laser processing lines, shown generally at 10 (which may also be referred to as "theoretical treatment lines") having a predetermined pixel spacing and determining the intersection(s) of the laser processing lines 10 with the weed-bounding polygon(s) generated in step S114. An example weed-bounding polygon 3a is illustrated in Figure 6(a). The laser processing lines 10 typically have a predetermined position within each acquired image by the camera 22 (i.e. independent of the weed positions), and are orientated in a substantially cross direction to the direction of motion.

Each laser processing line 10 extends across the full width W of the image and has a height dimension (perpendicular to the image width, i.e. along the direction of motion) that may be selected based on the desired precision and the resolution of the image. Typically, the height of the laser processing line is 10 image pixels or less, preferably 1-5 pixels. In preferred embodiments, each laser processing line has a height of 1 image pixel. In general, the dimension of each laser processing line 10 along the direction of motion is smaller than a dimension of the objects of interest for laser treatment (in image space).

The intersection(s) of the laser processing lines 10 with the polygon coordinates of the weed(s) are then determined in order to construct the laser treatment regions 12. The intersection points of the laser processing lines 10 with the polygon coordinates of the weeds is illustrated in Figure 6(b).

Thus, the laser processing lines 10 are divided into laser treatment region(s) 12 across which a laser is to be applied. This is schematically illustrated in Figure 6(c), where the highlighted laser processing line 10a is divided into two laterally spaced laser treatment regions 12a, 12b corresponding to the regions of the laser processing line 10a that overlap with the weed 3. This may also be referred to as a treatment line having two laterally spaced segments.

The laser treatment regions 12 are typically defined as coordinate pairs where the laser processing line 10 intersects with the weed-bounding polygons. For example, laser treatment regions 12a and 12b may be defined by an array of two intersection coordinate pairs [(x0, y0; x1, y1) (x0, y0; x1, y1)].

Following the determination of the laser treatment regions 12, at step S118 each laser treatment region 12 is converted from the image coordinate system (e.g. pixels) to the physical space coordinate system. This may be performed based on knowledge of the geometry of the camera 22 used to acquire the image relative to the ground. Laser treatment regions 12 within a subset of the laser processing lines 10 may then be processed by the laser at step S120.

Referring back to Figure 4(a), in the image I1, the single illustrated laser processing line (laser treatment line) 10a is processed by the laser. This line intersects with the identified weeds 3 in two laterally spaced laser treatment regions labelled 12a, 12b (e.g. segments of a laser treatment line). The laser therefore processes the two laser treatment regions 12a, 12b.

The laser beam is generated by the laser 24, with the direction of the laser beam controlled by a galvanometer scanner 26. Thus, the physical space coordinates are sent to the galvanometer scanner 26 with the one or more mirrors of the galvo scanner adjusted so as to treat the calculated treatment lines 12. The coordinates may be sent using a User Datagram Protocol (UDP) or other suitable communication protocol. If the laser processing line (or subset of laser processing lines) is determined to not intersect with any objects of interest for laser treatment, the laser is not activated for that image cycle.

In this example, within one cycle of steps S100 to S120, only one laser processing line 10a is processed and treated with the laser, as shown in Figure 4(a). This is in contrast to attempting to provide laser treatment of the whole area of each of the identified weeds 3 in the image I1 for example, which would take a significant amount of time. In this way, the time taken to perform each the sequence of steps S100 to S120 is minimised, and may be kept to 25ms or less. Following treatment of the laser treatment regions 12a, 12b in Figure 4(a), the cycle re-starts at S100 with the acquisition of a new image by camera 22, which is displaced compared to the first image. For example, at a movement speed of 300m/hr and a cycle time of 25ms, the second image will be displaced by ~2mm along the direction of movement compared to the first image.

The selected portion P2 of the second image is shown in Figure 4(b), where the agricultural crops 1 and weeds 3 have moved within the image frame relative to the first image. As shown in Figure 4(b), the equivalent laser processing line 10 now only intersects with one identified weed such that only one laser treatment 12 is processed for this cycle. The selected portion and laser processing line 10 are in the same relative position in each acquired image.

Therefore, by iteratively performing the cycle of steps S100 to S120 as described above, with only one laser processing line (or a subset of laser processing lines) being treated within a cycle ("per image"), the objects of interest may be laser treated whilst in continuous relative motion compared to the laser. Consequently, this allows for the present invention to provide laser treatment of the weeds while the apparatus 50 is continuously moving at speeds of up to 300m/hr (a typical average speed for manual weeding) with a ~2mm spatial error, which is deemed acceptable.

Figure 4(c) illustrates a portion of the next consecutive image obtained, where in this case the laser processing line 10 does not intersect with any identified weeds, and therefore no laser treatment is provided for this cycle.

Although in the presently described embodiment the laser treatment regions for a single laser processing line are treated with the laser beam in each cycle, in other embodiments, a subset of the laser processing lines (e.g. a subset of the laser processing lines shown in Figure 6(a)) are treated with the laser in a cycle. The above description describes constructing laser treatment regions using polygon coordinates of weeds. However, other techniques may be used to compare the locations of the laser processing lines and objects of interest, for example using pixel brightness techniques.

## Claims

1. A method, performed in a laser treatment apparatus (50), comprising:
(a) acquiring (S100) an image;
(b) identifying (S108) one or more objects (3) of interest for laser treatment within the acquired image;
(c) selecting (S102) a laser treatment area of the acquired image and dividing the laser treatment area into one or more laser processing lines (10);
(d) determining (S116) one or more laser treatment regions (12) within the laser processing line(s) based on the identified object(s) for laser treatment; and
(e) treating (S120) the laser treatment region(s) with a laser beam generated by a laser (24); wherein
step (a) is performed by a camera (22);
steps (b) to (d) are performed by an image processing unit (20);
**characterized in that**
the determining the one or more laser treatment regions within the laser processing line(s) comprises determining the regions where the objects of interest for laser treatment overlap with the laser processing line(s).

2. The method of claim 1, wherein the laser treatment region(s) within a subset of the processing lines are treated with the laser beam in step (e).

3. The method of any of claims 1 to 2, wherein the laser treatment region(s) within exactly one processing line are treated with the laser beam in step (e).

4. The method of any of claims 1 to 3, wherein each laser processing line is elongate and orientated in a cross direction to a direction of relative movement between the apparatus and the objects of interest.

5. The method of any of claims 1 to 4, comprising performing a plurality of the cycles of the steps (a) to (e) as the laser treatment apparatus and the objects of interest for laser treatment move relative to each other.

6. The method of any of claims 1 to 5, further comprising converting (S118) the determined laser treatment region(s) into physical space coordinates for treatment by the laser beam.

7. The method of any of claims 1 to 6, wherein the laser beam is directed by a galvanometer scanner.

8. The method of any of claims 1 to 7, wherein the identifying one or more objects for laser treatment within the acquired image comprises identifying objects of interest within the acquired image using a machine learning algorithm, preferably a neural network.

9. A laser treatment apparatus (50), comprising:
a camera (22) configured to acquire an image;
a laser (24) configured to generate a laser beam;
a laser beam positioning device (26) configured to direct the laser beam; and
an image processing unit (20) configured to perform the steps of:
(a) receive an image acquired by the camera;
(b) identify (S108) one or more objects of interest (3) for laser treatment within the acquired image;
(c) select (S102) a laser treatment area of the acquired image and divide the laser treatment area into one or more processing lines (10);
(d) determine (S116) one or more laser treatment regions (12) within the laser processing line(s) based on the identified object(s) for laser treatment; and
(e) control the laser and laser beam positioning device so as to treat (S120) the laser treatment regions(s) with the laser beam; wherein
the determining the one or more laser treatment regions within the laser processing line(s) comprises determining the regions where the objects of interest for laser treatment overlap with the laser processing line(s).

10. The laser treatment apparatus of claim 9, wherein the image processing unit is further configured to control the laser and laser beam positioning device such that the laser treatment region(s) within a subset of the processing lines are treated in step (e).

11. The laser treatment apparatus of claim 9 or claim 10, wherein the image processing unit is further configured to control the laser and laser beam positioning device such that the laser treatment region(s) within exactly one processing line are treated in step (e).

12. The laser treatment apparatus of any of claims 9 to 11, wherein the image processing unit is configured to perform the steps of any of claims 1 to 8.

13. The laser treatment apparatus of any of claims 9 to 12, wherein the laser is configured to generate a laser beam with a wavelength in the region of 400-500nm and a power density of at least 15kW / cm^2.

14. The laser treatment apparatus of any of claims 9 to 13, configured to be mounted on or integrated in a vehicle (1000) or a unit (100) configured to be attached to a vehicle (1000).

15. The method or laser treatment apparatus of any of the preceding claims, wherein the objects of interest for laser treatment are weeds or other unfavourable plants.

## Patentansprüche

1. Verfahren, das in einer Laserbehandlungsvorrichtung (50) durchgeführt wird, umfassend:
(a) Erfassen (S100) eines Bildes;
(b) Identifizieren (S108) eines oder mehrerer Objekte (3) innerhalb des erfassten Bildes, die für eine Laserbehandlung von Interesse sind;
(c) Auswählen (S102) eines Laserbehandlungsbereichs des erfassten Bildes und Aufteilen des Laserbehandlungsbereichs in eine oder mehrere Laserbearbeitungslinien (10);
(d) Bestimmen (S116) eines oder mehrerer Laserbehandlungsbereiche (12) innerhalb der Laserbearbeitungslinie(n) basierend auf dem/den identifizierten Objekt(en) zur Laserbehandlung;
und
(e) Behandeln (S120) des Bereichs /der Bereiche für Laserbehandlung mit einem von einem Laser (24) erzeugten Laserstrahl; wobei
Schritt (a) von einer Kamera (22) ausgeführt wird;
Schritte (b) bis (d) von einer Bildverarbeitungseinheit (20) ausgeführt werden;
**dadurch gekennzeichnet, dass** das Bestimmen der einen oder mehreren Laserbehandlungsbereiche innerhalb der Laserbearbeitungslinie(n) das Bestimmen der Bereiche umfasst, in denen sich die für die Laserbehandlung interessanten Objekte mit der / den Laserbearbeitungslinie(n) überlappen.

2. Verfahren nach Anspruch 1, wobei in Schritt (e) der / die Laserbehandlungsbereich(e) innerhalb eines Teilsatzes der Bearbeitungslinien mit dem Laserstrahl behandelt werden.

3. Verfahren nach einem der Ansprüche 1 bis 2, wobei in Schritt (e) der oder die Laserbehandlungsbereich(e) innerhalb genau einer Bearbeitungslinie mit dem Laserstrahl behandelt werden.

4. Verfahren nach einem der Ansprüche 1 bis 3, wobei jede Laserbearbeitungslinie länglich und quer zur Richtung der relativen Bewegung zwischen der Vorrichtung und den Objekten von Interesse ausgerichtet ist.

5. Verfahren nach einem der Ansprüche 1 bis 4, welches das Ausführen einer Vielzahl von Zyklen der Schritte (a) bis (e) umfasst, während sich die Laserbehandlungsvorrichtung und die Objekte von Interesse für die Laserbehandlung relativ zueinander bewegen.

6. Verfahren nach einem der Ansprüche 1 bis 5, das ferner das Umwandeln (S118) der ermittelten Laserbehandlungsregion(en) in physikalische Raumkoordinaten zur Behandlung durch den Laserstrahl umfasst.

7. Verfahren nach einem der Ansprüche 1 bis 6, wobei der Laserstrahl durch einen Galvanometer-Scanner gerichtet wird.

8. Verfahren nach einem der Ansprüche 1 bis 7, wobei das Identifizieren eines oder mehrerer Objekte zur Laserbehandlung innerhalb des erfassten Bildes das Identifizieren von Objekten von Interesse innerhalb des erfassten Bildes unter Verwendung eines maschinellen Lernalgorithmus, vorzugsweise eines neuronalen Netzwerks, umfasst.

9. Laserbehandlungsvorrichtung (50), umfassend:
Eine Kamera (22), die zur Erfassung eines Bildes konfiguriert ist;
einen Laser (24), der konfiguriert ist, einen Laserstrahl zu erzeugen;
eine Laserstrahlpositioniervorrichtung (26), die zum Lenken des Laserstrahls konfiguriert ist; und
eine Bildverarbeitungseinheit (20), die zur Ausführung der folgenden Schritte konfiguriert ist:
(a) Empfangen eines von der Kamera erfassten Bildes;
(b) Identifizieren (S108) eines oder mehrerer Objekte von Interesse (3) innerhalb des erfassten Bildes zur Laserbehandlung;
(c) Auswählen (S102) eines Laserbehandlungsbereichs des erfassten Bildes und Aufteilen des Laserbehandlungsbereichs in eine oder mehrere Bearbeitungslinien (10);
(d) Bestimmen (S116) eines oder mehrerer Laserbehandlungsbereiche (12) innerhalb der Laserbearbeitungslinie(n) basierend auf dem/den identifizierten Objekt(en) zur Laserbehandlung;
(e) Steuern des Lasers und der Laserstrahlpositioniervorrichtung, um die/den Laserbehandlungsbereich(e) mit dem Laserstrahl zu behandeln (S120); wobei
das Bestimmen des einen oder der mehreren Laserbehandlungsbereich(e) innerhalb der Laserbearbeitungslinie(n) das Bestimmen der Bereiche umfasst, in denen sich die Objekte von Interesse für die Laserbehandlung mit der / den Laserbearbeitungslinie(n) überlappen.

10. Laserbehandlungsvorrichtung nach Anspruch 9, wobei die Bildverarbeitungseinheit ferner dazu konfiguriert ist, den Laser und die Laserstrahlpositioniervorrichtung so zu steuern, dass in Schritt (e) der / die Laserbehandlungsbereich(e) innerhalb eines Teilsatzes der Bearbeitungslinien behandelt werden.

11. Laserbehandlungsvorrichtung nach Anspruch 9 oder Anspruch 10, wobei die Bildverarbeitungseinheit ferner dazu konfiguriert ist, den Laser und die Laserstrahlpositioniervorrichtung so zu steuern, dass in Schritt (e) der/die Laserbehandlungsbereich(e) innerhalb genau einer Bearbeitungslinie behandelt werden.

12. Laserbehandlungsvorrichtung nach einem der Ansprüche 9 bis 11, wobei die Bildverarbeitungseinheit so konfiguriert ist, dass sie die Schritte nach einem der Ansprüche 1 bis 8 ausführt.

13. Laserbehandlungsvorrichtung nach einem der Ansprüche 9 bis 12, wobei der Laser so konfiguriert ist, dass er einen Laserstrahl mit einer Wellenlänge im Bereich von 400-500 nm und einer Leistungsdichte von mindestens 15 kW/cm^2 erzeugt.

14. Laserbehandlungsvorrichtung nach einem der Ansprüche 9 bis 13, die so konfiguriert ist, dass sie an einem Fahrzeug (1000) montiert oder in dieses integriert werden kann, oder eine Einheit (100), die so konfiguriert ist, dass sie an einem Fahrzeug (1000) befestigt werden kann.

15. Verfahren oder Laserbehandlungsvorrichtung nach einem der vorhergehenden Ansprüche, wobei die Objekte, die für die Laserbehandlung von Interesse sind, Unkraut oder andere ungünstige Pflanzen sind.

## Revendications

1. Procédé, réalisé dans un appareil de traitement au laser (50), comprenant :
(a) l'acquisition (S100) d'une image ;
(b) l'identification (S108) d'un ou de plusieurs objets (3) d'intérêt pour un traitement au laser au sein de l'image acquise ;
(c) la sélection (S102) d'une zone de traitement au laser de l'image acquise et la division de la zone de traitement au laser en une ou plusieurs lignes de traitement au laser (10) ;
(d) la détermination (S116) d'une ou de plusieurs régions de traitement au laser (12) au sein de la ligne/des lignes de traitement au laser sur la base de l'objet/des objets identifié/s pour un traitement au laser ; et
(e) le traitement (S120) de la région/des régions de traitement au laser avec un faisceau laser généré par un laser (24) ; dans lequel
l'étape (a) est réalisée par une caméra (22) ;
les étapes (b) à (d) sont réalisées par une unité de traitement d'images (20) ;
**caractérisé en ce que**
la détermination des une ou plusieurs régions de traitement au laser au sein de la ligne/des lignes de traitement au laser comprend la détermination des régions où les objets d'intérêt pour un traitement au laser sont en chevauchement avec la ligne/les lignes de traitement au laser.

2. Procédé de la revendication 1, dans lequel la région/les régions de traitement au laser au sein d'un sous-ensemble des lignes de traitement sont traitées avec le faisceau laser dans l'étape (e).

3. Procédé selon n'importe lesquelles des revendications 1 à 2, dans lequel la région/les régions de traitement au laser située/s exactement au sein d'une ligne de traitement est/sont traitée/s avec le faisceau laser dans l'étape (e).

4. Procédé selon n'importe lesquelles des revendications 1 à 3, dans lequel chaque ligne de traitement au laser est allongée et orientée suivant une direction en croix vers une direction de mouvement relatif entre l'appareil et les objets d'intérêt.

5. Procédé selon n'importe lesquelles des revendications 1 à 4, comprenant la réalisation d'une pluralité des cycles des étapes (a) à (e) au fur et à mesure que l'appareil de traitement au laser et les objets d'intérêt pour un traitement au laser se déplacent relativement l'un à l'autre.

6. Procédé selon n'importe lesquelles des revendications 1 à 5, comprenant en outre la conversion (S118) de la région/des régions de traitement au laser déterminée/s en coordonnées spatiales physiques en vue d'un traitement par le faisceau laser.

7. Procédé selon n'importe lesquelles des revendications 1 à 6, dans lequel le faisceau laser est dirigé par un dispositif de balayage à galvanomètre.

8. Procédé selon n'importe lesquelles des revendications 1 à 7, dans lequel l'identification d'un ou de plusieurs objets pour un traitement au laser au sein de l'image acquise comprend l'identification d'objets d'intérêt au sein de l'image acquise grâce à l'utilisation d'un algorithme d'apprentissage machine, de préférence un réseau neuronal.

9. Appareil de traitement au laser (50), comprenant :
une caméra (22) configurée pour acquérir une image ;
un laser (24) configuré pour générer un faisceau laser ;
un dispositif de positionnement de faisceau laser (26) configuré pour diriger le faisceau laser ; et
une unité de traitement d'images (20) configurée pour réaliser les étapes consistant à :
(a) recevoir une image acquise par la caméra ;
(b) identifier (S108) un ou plusieurs objets d'intérêt (3) pour un traitement au laser au sein de l'image acquise ;
(c) sélectionner (S102) une zone de traitement au laser de l'image acquise et diviser la zone de traitement au laser en une ou plusieurs lignes de traitement (10) ;
(d) déterminer (S116) une ou plusieurs régions de traitement au laser (12) au sein de la ligne/des lignes de traitement au laser sur la base de l'objet/des objets identifié/s pour un traitement au laser ; et
(e) commander le laser et le dispositif de positionnement de faisceau laser de sorte à traiter (S120) la région/les régions de traitement au laser avec le faisceau laser ; dans lequel
la détermination des une ou plusieurs régions de traitement au laser au sein de la ligne/des lignes de traitement au laser comprend la détermination des régions où les objets d'intérêt pour un traitement au laser sont en chevauchement avec la ligne/les lignes de traitement au laser.

10. Appareil de traitement au laser de la revendication 9, dans lequel l'unité de traitement d'images est configurée en outre pour commander le laser et le dispositif de positionnement de faisceau laser de telle sorte que la région/les régions de traitement au laser au sein d'un sous-ensemble des lignes de traitement sont traitées dans l'étape (e).

11. Appareil de traitement au laser de la revendication 9 ou de la revendication 10, dans lequel l'unité de traitement d'images est configurée en outre pour commander le laser et le dispositif de positionnement de faisceau laser de telle sorte que la région/les régions de traitement au laser située/s exactement au sein d'une ligne de traitement est/sont traitée/s dans l'étape (e).

12. Appareil de traitement au laser de n'importe lesquelles des revendications 9 à 11, dans lequel l'unité de traitement d'images est configurée pour réaliser les étapes de n'importe lesquelles des revendications 1 à 8.

13. Appareil de traitement au laser de n'importe lesquelles des revendications 9 à 12, dans lequel le laser est configuré pour générer un faisceau laser avec une longueur d'ondes dans la région de 400-500 nm et une densité de puissance d'au moins 15 kW/cm².

14. Appareil de traitement au laser de n'importe lesquelles des revendications 9 à 13, configuré pour être monté sur ou intégré à un véhicule (1000) ou une unité (100) configurée pour être attachée à un véhicule (1000).

15. Procédé ou appareil de traitement au laser de n'importe lesquelles des revendications précédentes, dans lequel les objets d'intérêt pour un traitement au laser sont des mauvaises herbes ou d'autres plantes défavorables.
